# EUROPEAN PATENT APPLICATION

(11) **EP 3 382 716 A1**
(43) Date of publication of application: **03.10.2018**
(21) Application number: 18164981.5
(22) Date of filing: 29.03.2018
(51) Int. Cl.: G16H 50/30

(54) **INFORMATION PROCESSING DEVICE, INFORMATION PROCESSING METHOD, AND STORAGE MEDIUM**

(30) Priority: 30.03.2017 JP 2017067421
(71) Applicant: Tanita Corporation, Tokyo (JP)
(72) Inventor: YUKINO, Satsuki, Tokyo, Tokyo (JP)
(74) Representative: Barker Brettell LLP

(57) **Abstract**

An information processing device 2 includes a measured information obtaining unit 22, a fatigue element index computing unit 23, and an action improvement processing unit 25. The measured information obtaining unit 22 is configured to obtain life habit information on a life habit of a user. The fatigue element index computing unit 23 is configured to compute an element index regarding fatigue accumulation of the user in accordance with the life habit information obtained by the measured information obtaining unit 22. The action improvement processing unit 25 is configured to determine whether an improvement in living activity of the user is required or not on the basis of the element index computed by the fatigue element index computing unit 23.

## Description

### TECHNICAL FIELD

The present invention relates to information processing, and relates to an information processing device, an information processing method, and a storage medium that determine a health state of a user.

### BACKGROUND ART

As an information processing device, there has been proposed a body information prediction device that computes scores indicating degrees of activities such as an exercise, sleeping, and stress of a user to predict short-term changes in body information such as a weight or a proportion of skeletal muscle in accordance with the computed scores (see JP2016-31702A).

### SUMMARY OF INVENTION

The above-described device can predict the short-term changes in the body information. However, for example, in the case where a predicted value of the body information indicating a degree of obesity of the user whose life habit is disordered has a standard value, the life habit of the user is not evaluated.

Thus, it is sometimes difficult for the device that predicts the short-term changes in the body information to achieve improvement in living activity of the user who has a chronic health problem.

The present invention has been made focusing on such problem. An object of the present invention is to provide an information processing device, an information processing method, and a storage medium that achieve improvement in living activity of a user who has a chronic health problem.

According to a first aspect of the present invention, an information processing device includes obtaining means, computing means, and determining means. The obtaining means is configured to obtain life habit information on a life habit of a user. The computing means is configured to compute an element index regarding fatigue accumulation of the user in accordance with the life habit information obtained by the obtaining means. The determining means is configured to determine whether an improvement in living activity of the user is required or not on the basis of the element index computed by the computing means.

According to a second aspect, the obtaining means according to the first aspect is configured to further obtain attribute information on the user. The determining means is configured to change the element index or a determination threshold for the element index in accordance with the attribute information.

According to a third aspect, the computing means according to the first aspect or the second aspect is configured to compute an accumulation element index and a recovery element index as the element indexes. The accumulation element index contributes to the fatigue accumulation of the user. The recovery element index contributes to a recovery from fatigue of the user.

According to a fourth aspect, the determining means according to any one of the first aspect to the third aspect is configured to calculate a level of elimination of fatigue regarding the life habit in accordance with the accumulation element index and the recovery element index. The determining means is configured to determine whether the improvement in living activity is required or not on the basis of the level of elimination of fatigue.

According to a fifth aspect, the determining means according to any one of the first aspect to the fourth aspect is configured to weight the accumulation element index and the recovery element index in accordance with respective levels of contribution of the accumulation element index and the recovery element index that are contributing to the fatigue of the user.

According to a sixth aspect, the obtaining means according to any one of the first aspect to the fifth aspect is configured to obtain at least one of a sex, an age, and an occupation of the user as the attribute information. The determining means is configured to change the weighting in accordance with the attribute information.

According to a seventh aspect, the determining means according to any one of the first aspect to sixth aspect is configured to obtain a balance between a plurality of the element indexes regarding the fatigue accumulation. The determining means is configured to determine whether the improvement in living activity is required or not on the basis of the balance.

According to an eighth aspect, the determining means according to any one of the first aspect to the seventh aspect is configured to add each of the plurality of element indexes to calculate the level of elimination of fatigue caused by the life habit. The determining means is configured to determine whether the improvement in living activity is required or not on the basis of the level of elimination of fatigue and the balance.

According to a ninth aspect, the information processing device according to any one of the first aspect to the eighth aspect further includes notifying means configured to notify improvement information encouraging the improvement in living activity on the basis of a determination result by the determining means.

According to a tenth aspect of the present invention, a non-transitory computer-readable storage medium records a program to cause a computer configured to process life habit information on a life habit of a user to execute: an obtaining step of obtaining the life habit information; an computing step of computing an element index regarding fatigue accumulation of the user in accordance with the life habit information obtained by the obtaining; and a determining step of determining whether an improvement in living activity of the user is required or not on the basis of the element index computed by the computing.

According to an eleventh aspect of the present invention, an information processing method includes: an obtaining step of obtaining life habit information on a life habit of a user; an computing step of computing an element index regarding fatigue accumulation of the user in accordance with the life habit information obtained by the obtaining; and a determining step of determining whether an improvement in living activity of the user is required or not on the basis of the element index computed by the computing.

According to the aspects, the use of the element index regarding the fatigue accumulation of the user ensures grasping a fatigue trend of the user; therefore, the user who has a chronic health problem can improve the living activity.

### BRIEF DESCRIPTION OF DRAWINGS

FIG. 1 is a diagram illustrating an exemplary configuration of an information processing system that includes an information processing device in a first embodiment of the present invention;
FIG. 2 is a block diagram illustrating one example of a function configuration of the information processing device;
FIG. 3 is a flowchart illustrating a process procedure example regarding an information processing method in the embodiment;
FIG. 4 is a flowchart illustrating an example of a computing process that computes element indexes regarding fatigue accumulation of a user;
FIG. 5 is a flowchart illustrating one example of an evaluating process that evaluates a living activity in accordance with the element indexes;
FIG. 6 is a flowchart illustrating one example of a determining process that determines an overall level of fatigue in accordance with the element indexes;
FIG. 7 is a flowchart illustrating one example of the determining process to determine a fatigue balance in accordance with the element indexes;
FIG. 8 is a diagram describing health types specified on the basis of the overall level of fatigue and the fatigue balance;
FIG. 9 is a diagram illustrating one example of improvement information created for each health type;
FIG. 10A is a diagram illustrating one example of a display of the improvement information displayed by an information display device;
FIG. 10B is a diagram illustrating another example of the display of the improvement information displayed by the information display device;
FIG. 11 is a diagram illustrating a process procedure example regarding an information processing method of a second embodiment of the present invention;
FIG. 12 is a diagram describing one example of a method to determine a chronic fatigue state of a third embodiment of the present invention; and
FIG. 13 is a flowchart illustrating a process procedure example regarding the information processing method according to the embodiment.

### DESCRIPTION OF EMBODIMENTS

The following describes embodiments of the present invention with reference to the attached drawings.

### (First Embodiment)

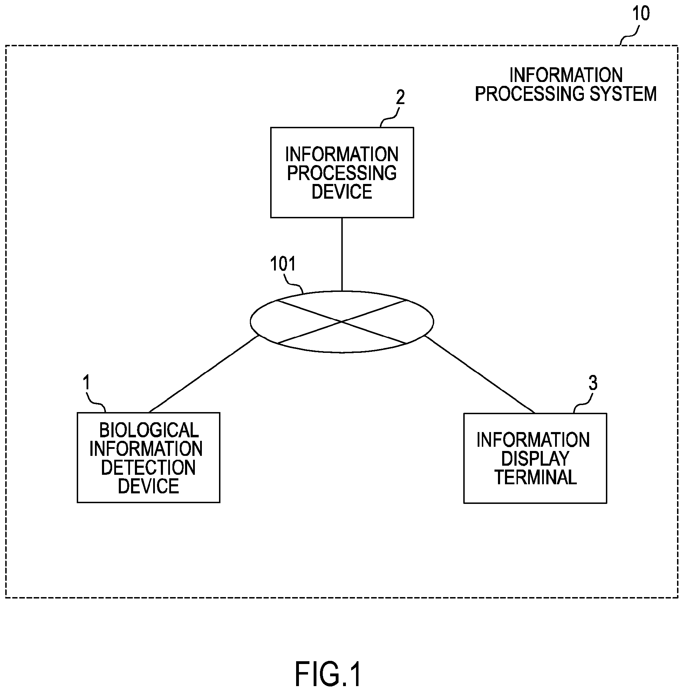

FIG. 1 is a diagram illustrating an exemplary configuration of an information processing system 10 in a first embodiment of the present invention.

The information processing system 10 includes a biological information detection device 1, an information processing device 2, and an information display terminal 3. The biological information detection device 1, the information processing device 2, and the information display terminal 3 in the information processing system 10 each communicates with one another over a network 101. The network 101 is, for example, constituted of a mobile phone network, a public telephone network, an Internet Protocol (IP) network, a wireless Local Area Network (LAN), and a similar network.

The biological information detection device 1 detects biological information on a health of a user as a measured person. The biological information includes, for example, biological information, which is obtained by directly measuring the user by the biological information detection device 1, biological information obtained through an input operation by the user, and biological information obtained by computing the biological information by a predetermined regression formula.

For example, the biological information detection device 1 is constituted of at least one of a plurality of measurement devices such as a body composition meter, an exhaled gas measurement device, an autonomic nerve measurement device, a sphygmomanometer, a sleep tracker, and an activity tracker. The biological information detection device 1 detects the biological information using the plurality of measurement devices to create life habit information on a life habit of the user in accordance with the detected biological information. The biological information detection device 1 may include a device that receives the biological information from one of or the plurality of measurement devices and creates the life habit information on the life habit of the user in accordance with the received biological information.

The life habit information includes, for example, an amount of meal, a meal balance, a level of fatigue, a quantity of exercise, a life rhythm, and a quality of sleep. The life habit information includes predetermined biological information on the life habit of the user itself, for example, a blood pressure value. The biological information detection device 1 transmits the biological information and the life habit information to the information processing device 2 over the network 101.

The information processing device 2 evaluates a health state of the user in accordance with the life habit information of the user. The information processing device 2 is a microcomputer that includes a Central Processing Unit (CPU) in which predetermined processes are programmed and a storage device. For example, the information processing device 2 is achieved by various mobile terminal devices such as a mobile phone, a Personal Digital Assistant (PDA), or a tablet PC (Personal Computer), a car navigation, or a server.

For example, the information processing device 2 receives the life habit information from the biological information detection device 1 and applies the received life habit information to, for example, the predetermined regression formula or a predetermined correspondence table for evaluation of a level of fatigue accumulation of the user. The information processing device 2 determines whether an improvement in living activity of the user is required or not on the basis of the evaluation value of the level of fatigue accumulation. The living activity here includes activities of a household, a job, and a hobby of the user in a day, the life habit, and a similar activity. The life habit includes habitual actions such as eating, an exercise, sleeping, smoking, drinking, and stress.

The information processing device 2 transmits the evaluation result regarding the level of fatigue accumulation, the determination result regarding the living activity, advice corresponding to the determination results, and similar information to the information display terminal 3 as action improvement information to improve the living activity of the user.

The information display terminal 3 receives the action improvement information from the information processing device 2, creates a display image on the basis of the action improvement information, and displays the display image in a screen. The information display terminal 3 is, for example, used by the user and is achieved by a mobile phone, a smart phone, a microcomputer, or a similar device.

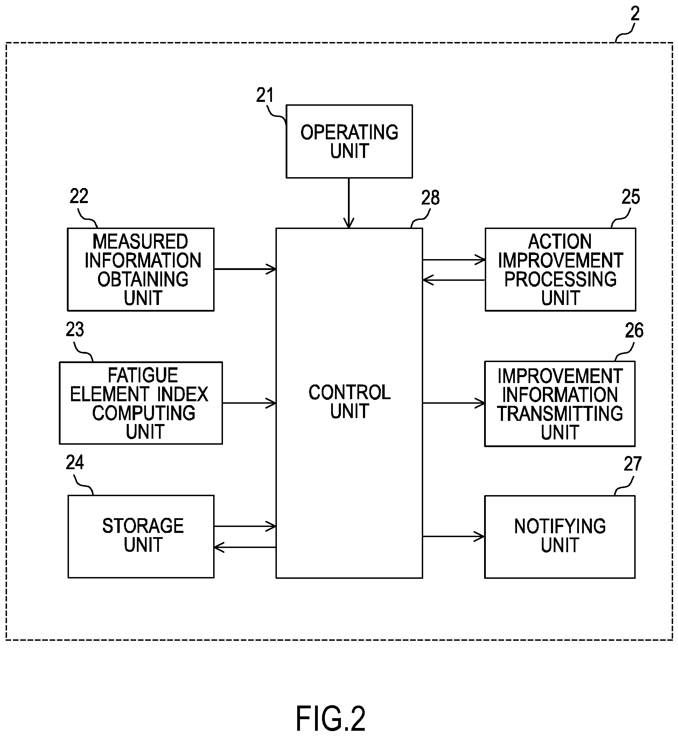

FIG. 2 is a block diagram illustrating one example of a function configuration of the information processing device 2 of the embodiment.

The information processing device 2 includes an operating unit 21, a measured information obtaining unit 22, a fatigue element index computing unit 23, a storage unit 24, an action improvement processing unit 25, an improvement information transmitting unit 26, a notifying unit 27, and a control unit 28.

The operating unit 21 includes, for example, a touch sensor, a keyboard, and a computer mouse and accepts the information input by a user operation using these devices. For example, in the case where the information required for the determination on the living activity is insufficient, the operating unit 21 accepts the information on the user by the input operation.

The measured information obtaining unit 22 constitutes obtaining means that obtains the life habit information of the user. The measured information obtaining unit 22 of this embodiment receives the life habit information transmitted from the biological information detection device 1.

The fatigue element index computing unit 23 constitutes computing means that computes a fatigue element index regarding a trend of the fatigue accumulation (an increasing trend) of the user in accordance with the life habit information of the user. The fatigue element index computing unit 23 of this embodiment calculates respective recovery element index contributing to recovery from fatigue and an accumulation element index contributing to the accumulation of the fatigue in accordance with the life habit information of the user.

The above-described recovery element index includes, for example, an energy intake content, which is an index evaluating the recovery from fatigue caused by the meal taken by the user, and fatigue recovery ability, which is an index evaluating the recovery from fatigue caused by the sleeping by the user. The evaluation values of the recovery element indexes of the embodiment have a trend to increase as the user eliminates the fatigue accumulated in the user.

The accumulation element index includes, for example, a physiological fatigue level, which is an index evaluating the fatigue accumulated by the exercise by the user, and a mental fatigue level, which is an index evaluating the mental fatigue. As the user accumulates the fatigue, the fatigue is less likely to be eliminated; therefore, the evaluation values of the accumulation element indexes of this embodiment have a trend to become low as the user accumulates the fatigue.

Thus, the respective fatigue element indexes such as the above-described physiological fatigue level, mental fatigue level, energy intake content, and fatigue recovery ability are calculated so as to be normalized such that the fatigue element indexes can be uniformly treated mutually.

The storage unit 24 constitutes storage means that stores the action improvement information corresponding to the determination result of the living activity. The storage unit 24 also stores the life habit information obtained by the operating unit 21 and the measured information obtaining unit 22.

The storage unit 24 is constituted of a non-volatile memory (Read Only Memory: ROM), a volatile memory (Random Access Memory: RAM), and a similar memory. The storage unit 24 stores a control program to control the behaviors of the information processing device 2. That is, the storage unit 24 is a storage medium storing the programs to achieve the functions of this embodiment.

The action improvement processing unit 25 constitutes determining means that determines whether the improvement in living activity of the user is required or not on the basis of the fatigue element indexes computed by the fatigue element index computing unit 23. The determining means here includes, not only one determining whether the improvement in living activity of the user is required or not but also one that performs a computing process required for the determination.

For example, the action improvement processing unit 25 adds the recovery element indexes to the accumulation element indexes among the fatigue element indexes to calculate a level of elimination of fatigue (a fatigue elimination level) that indicates an overall level of elimination of different fatigues. This level of elimination of fatigue is likely to increase as the accumulated fatigue of the user decreases. When the level of elimination of fatigue is less than a total threshold, the action improvement processing unit 25 determines that the improvement in living activity of the user is required. The total threshold is preliminarily fixed taking statistical data, experimental data, or similar data into consideration.

Alternatively, the action improvement processing unit 25 may calculate the fatigue balance that indicates a degree of balance between at least the two fatigue element indexes among the physiological fatigue and the mental fatigue included in the accumulation element index, and the energy intake content and the fatigue recovery ability included in the recovery element index. With the fatigue balance exceeding a balance threshold, the action improvement processing unit 25 determines that the living activity needs to be improved. The balance threshold is preliminarily fixed taking the statistical data, the experimental data, or similar data into consideration similar to the total threshold.

The action improvement processing unit 25 creates the action improvement information indicative of the necessity of the improvement in living activity according to identification information to specify the user, attribute information indicative of a sex, an age, and similar information of the user, the values of the respective fatigue element indexes, the value of the level of fatigue accumulation, the value of the fatigue balance, and a similar value. The action improvement processing unit 25 causes the storage unit 24 to store the created action improvement information.

To encourage the user to improve the living activity, the improvement information transmitting unit 26 transmits the action improvement information stored in the storage unit 24 to the information display terminal 3 over the network 101 illustrated in FIG. 1.

The notifying unit 27 notifies the necessity of the improvement in living activity of the user. For example, to manage the health states of the plurality of users, the notifying unit 27 displays the action improvement information for each piece of identification information of the user.

When it is determined that the improvement in living activity is required, the notifying unit 27 may flash a lamp and generate a rumbling sound. It should be noted that the notifying unit 27 may create the action improvement information on the basis of the values of the respective fatigue element indexes.

The control unit 28 is constituted of the central processing unit, an input interface, and a bus mutually coupling these units. The control unit 28 reads the control program stored in the storage unit 24 and causes the central processing unit to execute the control program to control the respective units in the information processing device 2 via the input interface. Alternatively, the central processing unit constituting the control unit 28 may function as the measured information obtaining unit 22, the fatigue element index computing unit 23, the action improvement processing unit 25, the improvement information transmitting unit 26, and a similar unit.

The control unit 28 of the embodiment controls the respective operating unit 21, measured information obtaining unit 22, fatigue element index computing unit 23, storage unit 24, action improvement processing unit 25, improvement information transmitting unit 26, and notifying unit 27.

The control unit 28 obtains the life habit information from the operating unit 21 and the measured information obtaining unit 22, controls the fatigue element index computing unit 23 so as to compute the fatigue element indexes in accordance with the life habit information, and controls the action improvement processing unit 25 so as to determine whether the improvement in living activity is required or not on the basis of the fatigue element indexes.

The control unit 28 extracts the action improvement information from the storage unit 24 according to the determination result by the action improvement processing unit 25. Further, the control unit 28 displays the action improvement information in the notifying unit 27, and transmits the action improvement information to the information display terminal 3 via the improvement information transmitting unit 26.

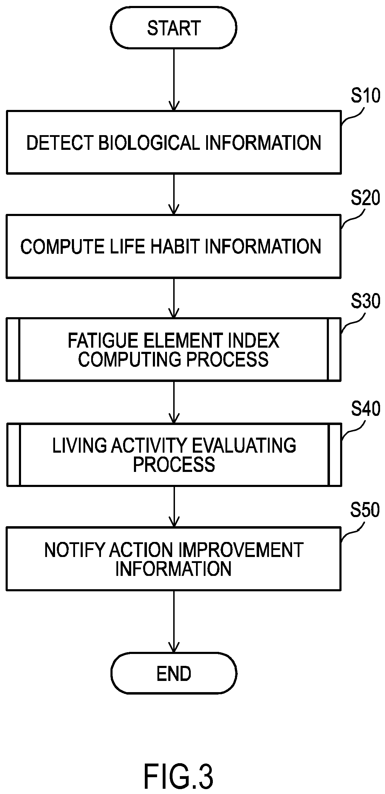

FIG. 3 is a flowchart illustrating a process procedure example regarding the information processing method by the information processing system 10.

At Step S10, the biological information detection device 1 detects the biological information using the measurement devices such as the body composition meter, the exhaled gas measurement device, a stress meter, the sphygmomanometer, the sleep tracker, and the activity tracker. Further, the biological information detection device 1 obtains attribute information indicative of information such as the sex, the age, and an occupation of the user through the input operation of the user as the biological information.

At Step S20, the biological information detection device 1 uses the detected and obtained biological information to compute the life habit information on the life habit of the user.

For example, the biological information detection device 1 uses the measurement devices such as the body composition meter and the exhaled gas measurement device to evaluate the amount of meal and the meal balance and uses the measurement devices such as the autonomic nerve measurement device, the sphygmomanometer, and the sleep tracker to evaluate the level of fatigue. Further, the biological information detection device 1 uses the measurement devices such as the activity tracker to evaluate the quantity of exercise, the life rhythm, and the quality of sleep.

The biological information detection device 1 thus uses the plurality of measurement devices to evaluate each of the amount of meal, meal balance, level of fatigue, quantity of exercise, life rhythm, and quality of sleep. The biological information detection device 1 transmits the life habit information in addition to the respective evaluated values to the information processing device 2 together with the attribute information. The life habit information indicates the biological information required to calculate the fatigue element indexes.

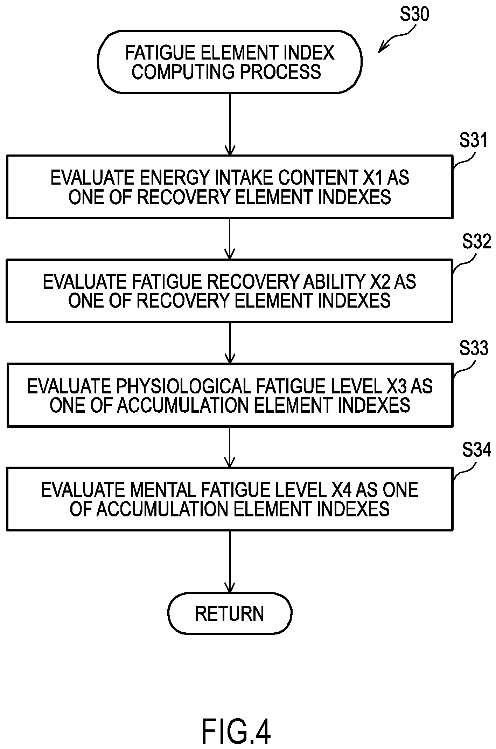

At Step S30, the information processing device 2 executes a fatigue element index computing process that computes the fatigue element indexes in accordance with the life habit information. This fatigue element index computing process will be described later with reference to FIG. 4.

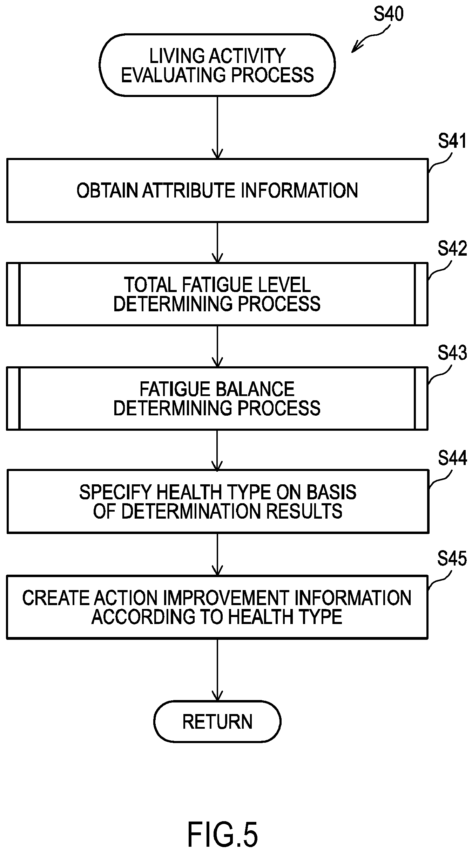

At Step S40, the information processing device 2 executes a living activity evaluating process that determines whether the improvement in living activity is required or not on the basis of the fatigue element indexes. This living activity evaluating process will be described later with reference to FIG. 5. The information processing device 2 creates the action improvement information according to the determination result and transmits the action improvement information to the information display terminal 3.

At Step S50, the information display terminal 3 receives the action improvement information from the information processing device 2 and notifies a signal encouraging the improvement in living activity of the user on the basis of the received action improvement information. The information display terminal 3 of this embodiment displays the evaluation result regarding the level of elimination of fatigue included in the action improvement information, the determination result regarding the living activity, and the advice corresponding to these results in the screen.

When the process at Step S50 is terminated, a sequence of the process procedures of the information processing method by the information processing system 10 are terminated. It should be noted that, while this embodiment calculates the life habit information by the biological information detection device 1, the information processing device 2 may calculate the life habit information on the basis of the biological information from the biological information detection device 1. The storage unit 24 in the information processing device 2 at least stores the programs of the processes at Steps S20 to S40 executable by the computer.

FIG. 4 is a flowchart illustrating a process procedure example regarding the fatigue element index computing process at Step S30.

At Step S31, the fatigue element index computing unit 23 in the information processing device 2 uses the meal information such as the amount of meal and the meal balance among the life habit information to evaluate the energy intake content X1 as the recovery element index.

For example, the fatigue element index computing unit 23 extracts values such as an intake of a nutrient and an intake of a calorie required for the recovery from fatigue, the muscle repair, and a similar purpose among the meal information and applies these values to the predetermined regression formula or the predetermined correspondence table to calculate the energy intake content X1. The nutrient required for the recovery from fatigue includes, for example, a protein having an amino acid. Thus, the energy intake content X1 is an index that comprehensively evaluates, for example, whether the nutrients required for the recovery from fatigue and the muscle repair are taken in a balanced manner.

The above-described meal information is obtained by, for example, analyzing a meal image taken by the information display terminal 3. Specifically, the biological information detection device 1 preliminarily stores dictionary data regarding the plurality of meal images. When the biological information detection device 1 receives the meal image from the information display terminal 3, the biological information detection device 1 performs image analysis on the meal image and extracts the feature value from the meal image. The biological information detection device 1 specifies a meal image with the feature value of the highest match with the extracted feature value among the feature values of the plurality of meal images included in the dictionary data. The biological information detection device 1 extracts the kind of the amino acid corresponding to the specified meal image, the amount of the protein, and the amount of the calorie from the dictionary data to create the meal information.

At Step S32, the fatigue element index computing unit 23 uses the quality of sleep among the life habit information to evaluate the fatigue recovery ability X2 as the recovery element index. For example, the fatigue element index computing unit 23 uses the predetermined regression formula or the predetermined correspondence table to convert an evaluation value regarding the quality of sleep into the fatigue recovery ability X2. For example, the higher the evaluation value regarding the quality of sleep, the larger the fatigue recovery ability X2 is.

The above-described quality of sleep can be obtained in accordance with, for example, the sleep tracker of the biological information detection device 1. The sleep tracker is constituted of a body motion sensor. The sleep tracker detects the body motion of the user, the vibrations caused by respiration and pulses, and a similar factor and the sleep tracker calculates the period of sleeping, the depth of sleeping, the rhythm of sleeping, and a similar factor to evaluate the quality of sleep from these values.

At Step S33, the fatigue element index computing unit 23 uses the quantity of exercise, the life rhythm, the blood pressure value, or a similar factor among the life habit information to evaluate a physiological fatigue level X3 as an accumulation element index. For example, the fatigue element index computing unit 23 applies the values such as the quantity of exercise, the life rhythm, and the blood pressure value to the predetermined regression formula or the predetermined correspondence table to calculate the physiological fatigue level X3. Thus, the physiological fatigue level X3 is an index that comprehensively evaluates excessive exercise, the disorder of life rhythm, and a similar factor.

The above-described quantity of exercise and life rhythm can be obtained in accordance with, for example, the activity tracker of the biological information detection device 1. The activity tracker is constituted of an acceleration sensor or a similar sensor. The activity tracker calculates the quantity of exercise of the user on the basis of a sum of the detected values by the acceleration sensor mounted to the user and the activity tracker specifies the activity pattern in a day using the quantity of exercise to evaluate the life rhythm.

At Step S34, the fatigue element index computing unit 23 uses, for example, a Low-Frequency/High-Frequency (LF/HF), which is an index that indicates the amount of variation of the blood pressure value and activities of an autonomic nervous system among the life habit information, to evaluate a mental fatigue level X4 as the accumulation element index. For example, the fatigue element index computing unit 23 applies the respective values of the amount of variation of the blood pressure value and LF/HF to the predetermined regression formula or the predetermined correspondence table to calculate the mental fatigue level X4. Thus, the mental fatigue level X4 is an index that comprehensively evaluates an environment such as a workplace, presence/absence of a buffering factor such as family, and a similar factor.

The above-described LF/HF is a power ratio between an LF band that indicates the degree of activity of both sympathetic and parasympathetic systems and an HF band that indicates the degree of activity of the parasympathetic system. The LF/HF can be obtained from a heartbeat sensor, a pulse wave sensor, or a similar sensor of the biological information detection device 1 or a similar sensor.

When the process at Step S34 is terminated, the control unit 28 terminates a sequence of the process procedures of the fatigue element index computing process at Step S30 and returns the process to the process procedure of the control method shown in FIG. 3 to execute the living activity evaluating process at Step S40.

FIG. 5 is a flowchart illustrating the process procedure example regarding the living activity evaluating process at Step S40.

At Step S41, for evaluation of the living activity, the action improvement processing unit 25 in the information processing device 2 obtains the attribute information of the user in order to take differences in the sex, the age, the occupation, and a similar factor of the user into consideration. For example, occupations whose working hours are irregular, such as a nurse, a person engaged in shiftwork, and a long-distance driver, are likely to accumulate the fatigue compared with occupations of regular working hours where desk work is the main work. Therefore, the use of the attribute information of the user allows the evaluation taking the work contents of the user into consideration.

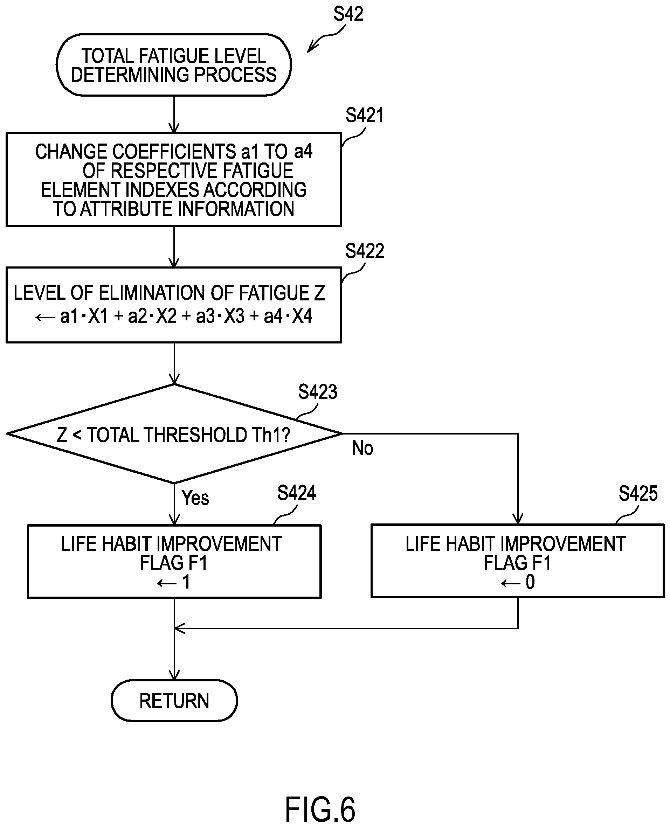

At Step S42, the action improvement processing unit 25 uses the respective fatigue element indexes X1 to X4 computed at Step S30 to execute a total fatigue level determining process that determines the overall level of fatigue of the user. This total fatigue level determining process will be described later with reference to FIG. 6.

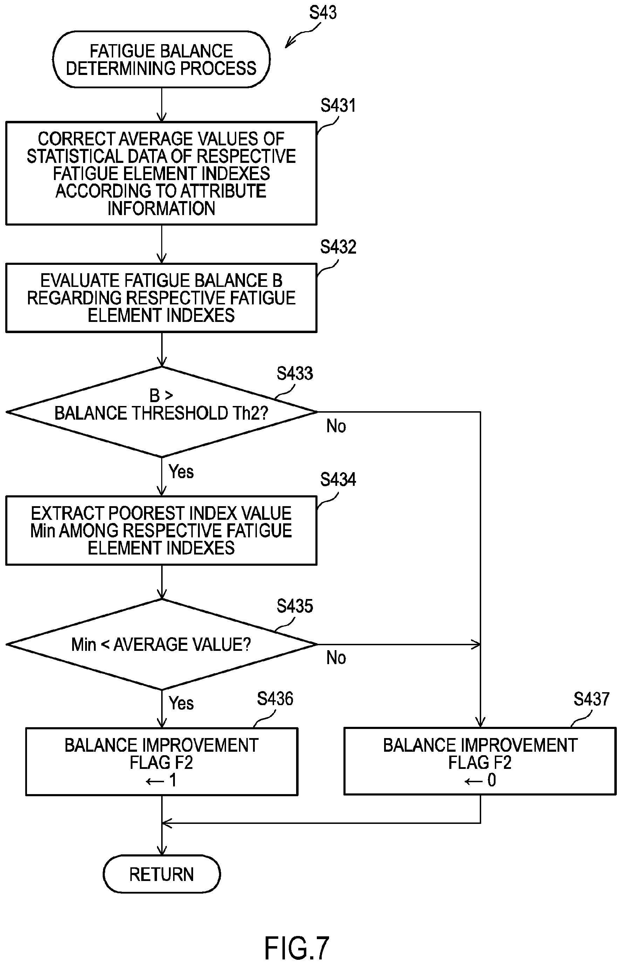

At Step S43, the action improvement processing unit 25 executes a fatigue balance determining process that determines the balance between the respective fatigue element indexes. This fatigue balance determining process will be described later with reference to FIG. 7.

At Step S44, the action improvement processing unit 25 determines a health type on the basis of both determination results at the respective processes at Steps S42 and S43.

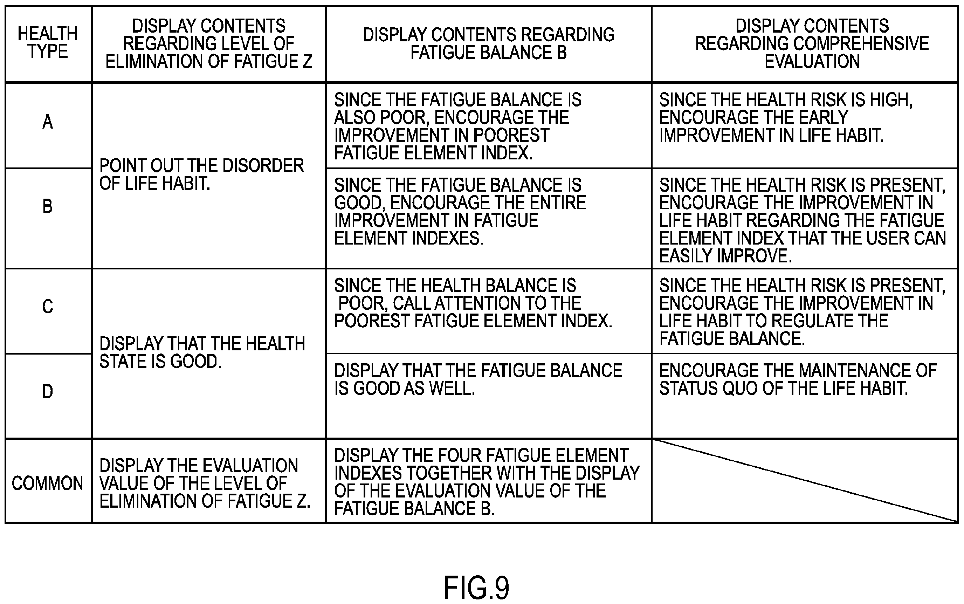

At Step S45, the action improvement processing unit 25 creates the action improvement information on the basis of the health type specified at Step S44 and stores the action improvement information in the storage unit 24. The action improvement information will be described later with reference to FIG. 9.

When the process at Step S45 is terminated, the control unit 28 terminates a sequence of the process procedures of the living activity evaluating process at Step S40 and returns the process to the process procedure of the control method shown in FIG. 3 to advance the process to a process at Step S50.

FIG. 6 is a flowchart illustrating a process procedure example regarding the total fatigue level determining process at Step S42. In this example, as a health index indicating the level of fatigue caused by the disorder of life habit of the user, a level of elimination of fatigue Z is computed.

At Step S421, the action improvement processing unit 25 calculates coefficients a1 to a4 multiplied to the respective fatigue element indexes of the energy intake content X1, the fatigue recovery ability X2, the physiological fatigue level X3, and the mental fatigue level X4 according to the attribute information obtained at Step S41.

With this embodiment, the storage unit 24 preliminarily stores reference values of the coefficients a1 to a4 fixed on the basis of the levels of contribution contributing to the fatigue of the user. It should be noted that the reference values of the coefficients a1 to a4 are fixed such that the level of elimination of fatigue Z is normalized in a value range of, for example, 0 to 100.

For example, in the case where the attribute information shows the occupation whose working hours are irregular and therefore the life habit is likely to be irregular, since the fatigue is likely to accumulate and eliminating the fatigue is difficult compared with the occupations with the regular working hours, the physiological fatigue level X3 is likely to lower. Taking such property into consideration, the action improvement processing unit 25 sets the coefficient a3 of the physiological fatigue level X3 to a value smaller than the reference value to avoid the determination result too strict with the user.

As the age shown in the attribute information higher than the twenties, the fatigue recovery ability X2 is likely to lower. Accordingly, the action improvement processing unit 25 sets the coefficient a2 of the fatigue recovery ability X2 to a value larger than the reference value to avoid the determination result to be too strict. Alternatively, the higher a post in a company, the fatigue is likely to accumulate and is difficult to be eliminated; therefore, the mental fatigue level X4 is likely to lower. In view of this, the action improvement processing unit 25 may set the coefficient a4 of the mental fatigue level X4 to a value smaller than the reference value as the post indicated by the attribute information becomes high.

Thus, since the standard level on the level of elimination of fatigue Z changes depending on the differences in attributes such as the sex, the age, the occupation, and a similar factor of the user, the action improvement processing unit 25 weights the respective fatigue element indexes according to the attribute information on the user. That is, the action improvement processing unit 25 changes the fatigue element indexes X1 to X4 according to the attribute information of the user. Thus, a physical property and a labor environment of the user and a similar factor are reflected to the level of elimination of fatigue Z, ensuring encouraging the improvement in realistic life habit reasonable for the user.

At Step S422, the action improvement processing unit 25 multiplies the respective coefficients a1 to a4 calculated at Step S421 to the fatigue element indexes X1 to X4 and sets the sum of the multiplied values to the level of elimination of fatigue Z. Thus, the action improvement processing unit 25 comprehensively evaluates the plurality of factors constituting the fatigue of the user to calculate the level of elimination of fatigue Z having a correlation with the degree of the disorder of life habit by the user.

At Step S423, the action improvement processing unit 25 determines whether the level of elimination of fatigue Z falls below the above-described total threshold Th1 or not. That is, the action improvement processing unit 25 determines whether the fatigue of the user is likely to accumulate caused by the disorder of life habit or not.

The total threshold Th1 is a threshold to determine whether the life habit is disordered or not. The total threshold Th1 is, for example, set to a statistic such as an average value, a median, or a mode of the level of elimination of fatigue Z or a value smaller than the statistic. This embodiment sets the total threshold Th1 to "50."

At Step S424, when the level of elimination of fatigue Z falls below the total threshold Th1, since the fatigue is likely to accumulate, the action improvement processing unit 25 determines that the life habit is disordered and sets a life habit improvement flag F1 to "1."

At Step S425, when the level of elimination of fatigue Z is equal to or more than the total threshold Th1, the action improvement processing unit 25 determines that the life habit is not disordered and sets the life habit improvement flag F1 to "0."

When the process at Step S424 or S425 is terminated, the control unit 28 terminates a sequence of the process procedures of the total fatigue level determining process at Step S42 and returns the process to the process procedure of the living activity evaluating process shown in FIG. 5 to advance the process to a process at Step S43.

It should be noted that while this embodiment changes the fatigue element indexes X1 to X4 according to the attribute information of the user, the total threshold Th1 may be changed according to the attribute information. For example, in the case where the attribute information indicates the occupation of irregular working hours, since the level of elimination of fatigue Z is likely to lower, the action improvement processing unit 25 reduces the total threshold Th1.

Specifically, the storage unit 24 preliminarily stores a threshold table indicative of the total thresholds fixed for each occupation. When the action improvement processing unit 25 obtains the attribute information of the user, the action improvement processing unit 25 refers to the threshold table and obtains the total threshold corresponding to the occupation indicated by the attribute information in the threshold table. This allows encouraging the improvement in realistic life habit by the simple computing process.

FIG. 7 is a flowchart illustrating the process procedure example regarding the fatigue balance determining process at Step S43. In this example, the storage unit 24 preliminarily stores the average values of the respective fatigue element indexes X1 to X4 obtained from the statistical data. The statistical data shows a distribution of the number of persons in the respective value ranges of the fatigue element indexes.

At Step S431, the action improvement processing unit 25 obtains the respective average values of the statistical data regarding the energy intake content X1, the fatigue recovery ability X2, the physiological fatigue level X3, and the mental fatigue level X4 and changes the average values of the respective fatigue element indexes according to the attribute information at Step S41.

For example, when the attribute information of the user indicates the occupation of the irregular working hours, the value of the physiological fatigue level X3 is likely to be lower than the average value thereof. In view of this, the action improvement processing unit 25 changes the average value of the physiological fatigue level X3 to a value smaller than the average value in the storage unit 24.

At Step S432, the action improvement processing unit 25 evaluates a fatigue balance B regarding the four fatigue element indexes X1 to X4.

The action improvement processing unit 25 of this embodiment calculates respective multiplication value of the energy intake content X1 and the fatigue recovery ability X2, multiplication value of the fatigue recovery ability X2 and the physiological fatigue level X3, multiplication value of the physiological fatigue level X3 and the mental fatigue level X4, and multiplication value of the mental fatigue level X4 and the energy intake content X1. The action improvement processing unit 25 calculates a variance value regarding the calculated four multiplication values as the fatigue balance B.

In view of this, the larger the variation between the four fatigue element indexes X1 to X4, that is, the poorer the balance between the four fatigue element indexes X1 to X4, the evaluation value of the fatigue balance B becomes large. Thus, the use of the above-described variance value as the fatigue balance B ensures grasping whether a specific index stands out compared with the other indexes among the four fatigue element indexes X1 to X4 or not.

At Step S433, the action improvement processing unit 25 determines whether the fatigue balance B exceeds a balance threshold Th2 or not. The balance threshold Th2 is a threshold to determine whether the index indicative of the poor state is present or not among the four fatigue element indexes X1 to X4. For example, the balance threshold Th2 is set in accordance with the statistic such as the average value, the median, or the mode of the fatigue balance B as the reference.

That is, the action improvement processing unit 25 determines whether at least one of the indexes among the fatigue element indexes X1 to X4 possibly becomes poor or not.

For example, when only the energy intake content X1 is higher than the average value and the other fatigue recovery ability X2, physiological fatigue level X3, and mental fatigue level X4 all have values lower than the average values, there is a possibility that the irregular meals and a loss of appetite result in the poor energy intake content X1. Thus, in the case where only the one fatigue element index is good and the other fatigue element indexes are all poor, there is a risk of making the satisfactory fatigue element index poor.

Alternatively, when only the fatigue recovery ability X2 and the physiological fatigue level X3 are good while the energy intake content X1 and the mental fatigue level X4 are poor, the poor quality of sleep and the reduction in quantity of exercise possibly result in any one of the fatigue recovery ability X2 and the physiological fatigue level X3 becoming poor. Alternatively, with the poor mental fatigue level X4, even if the other fatigue element indexes X1 to X3 are good, this possibly adversely affects any of the fatigue element indexes.

As described above, it is assumed that in the case where the specific fatigue element index is poor, the satisfactory fatigue element index becomes poor and the level of elimination of fatigue Z becomes poorer. In view of this, the evaluation of the variation of the fatigue balance B ensures a prediction whether the level of elimination of fatigue Z becomes poorer or not.

It should be noted that when the one fatigue element index is good, even if the other fatigue element indexes are poor, the fatigue element index possibly provides good influence to any of the fatigue element indexes. For example, when the mental fatigue level X4 to which an external factor such as the working environment acts is good, even if the other fatigue element indexes X1 to X3 are poor, the mental fatigue level X4 possibly provides the good influence to any of the fatigue element indexes. In view of this, when the mental fatigue level X4 is good among the four fatigue element indexes, the action improvement processing unit 25 may correct the evaluation value of the fatigue balance B to be a small value so as to make the level of elimination of fatigue Z better.

At Step S434, when the fatigue balance B exceeds the balance threshold Th2, the action improvement processing unit 25 determines that the fatigue balance B is poor and extracts a value Min of the poorest index among the values of the respective fatigue element indexes. For example, the action improvement processing unit 25 calculates differences found by subtracting the average values from the values of the fatigue element indexes for each fatigue element index to obtain the minimum value from the calculated differences, that is, the smallest value among the four fatigue element indexes X1 to X4, as the value Min.

At Step S435, when the value Min of the poorest index falls below the average value after the correction at Step S431, the action improvement processing unit 25 determines that the level of elimination of fatigue Z possibly becomes poorer and sets a balance improvement flag F2 to "1."

Meanwhile, at Step S436, when the fatigue balance B is equal to or less than the balance threshold Th2 or when the value Min of the poorest index is equal to or more than the average value, since the necessity to improve the fatigue balance B is low, the action improvement processing unit 25 sets the balance improvement flag F2 to "0."

When the process at Step S436 or S437 is terminated, the control unit 28 terminates a sequence of the process procedures of the fatigue balance determining process at Step S42 and returns the process to the process procedure of the living activity evaluating process shown in FIG. 5 to advance the process to the process at Step S43.

It should be noted that while in this embodiment, the variance value regarding the multiplication values of the fatigue element indexes different from one another as the fatigue balance B is calculated, this should not be construed in a limiting sense. It is only necessary that the variations between the respective fatigue element indexes can be grasped.

For example, the action improvement processing unit 25 may calculate average value found by averaging the values of the four fatigue element indexes and obtains difference between the value of the fatigue element index and the average value for each fatigue element index to calculate the maximum value out of absolute values of the obtained differences as the fatigue balance B. Alternatively, the action improvement processing unit 25 may calculate difference absolute value between the value of the fatigue element index and the statistics of the fatigue element index for each fatigue element index to calculate the sum of the respective difference absolute values as the fatigue balance B.

While in this embodiment, the fatigue balance B is evaluated by the two levels, good and poor, the action improvement processing unit 25 may classify the fatigue balance B into four levels, excellent, good, fair, and poor. For example, when the fatigue balance B is correlated to a degeneration risk of the fatigue element index, the fatigue balance may be classified into a plurality of levels according to the degree of correlation. Further, in the flowchart shown in FIG. 7, when the fatigue balance B exceeds the balance threshold Th2 at Step S433, the action improvement processing unit 25 may omit the processes at Steps S434 and Step S435 and may set the balance improvement flag F2 to "1."

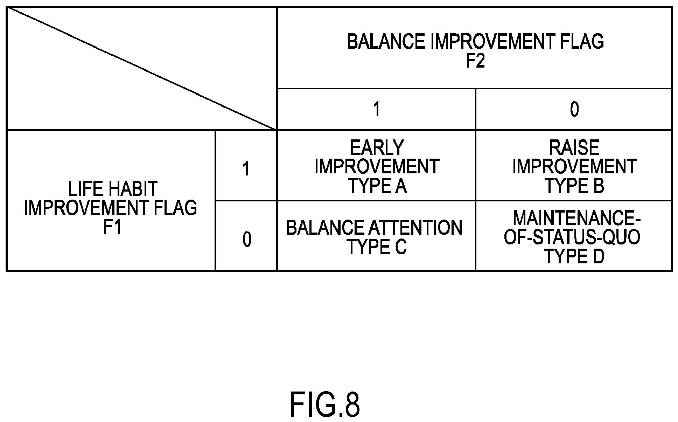

FIG. 8 is a diagram describing a relationship between the determination results of the level of elimination of fatigue Z and the fatigue balance B and the health types of the user.

When the life habit improvement flag F1 indicates "1" and the balance improvement flag F2 indicates "1," the life habit of the user is disordered and the risk of further degenerating the health state is high. In view of this, the action improvement processing unit 25 sets the health type of the user to an early improvement type A.

When the life habit improvement flag F1 indicates "1" and the balance improvement flag F2 indicates "0," although the life habit of the user is disordered, the risk of further degenerating the health state is not so high. In view of this, the action improvement processing unit 25 sets the health type to a raise improvement type B.

When the life habit improvement flag F1 indicates "0" and the balance improvement flag F2 indicates "1," although the life habit of the user is not disordered, there is a risk of degenerating the health state. In view of this, the action improvement processing unit 25 sets the health type to a balance attention type C.

When the life habit improvement flag F1 indicates "0" and the balance improvement flag F2 indicates "0," the life habit of the user is not disordered and the fatigue is less likely to accumulate. In view of this, the action improvement processing unit 25 sets the health type of the user to a maintenance-of-status-quo type D.

Thus, the action improvement processing unit 25 determines the health type of the user on the basis of the determination results of the level of elimination of fatigue Z and the fatigue balance B.

FIG. 9 is a diagram describing one example of the action improvement information created for each health type.

With the health type of the early improvement type A and the raise improvement type B, information indicative of the disorder of life habit is stored in the action improvement information.

With the health type of the early improvement type A, since the risk of degenerating the health state is high, information encouraging the improvement in poorest fatigue element index is stored in the action improvement information, in addition to the information indicative of the necessity of the early improvement in life habit.

Meanwhile, with the health type of the raise improvement type B, since there is a risk of degenerating the health state, information proposing reform measures for the life habit that the user easily tackles with is stored together with the information encouraging the overall improvement in respective fatigue element indexes of the life habit in the action improvement information. For example, to achieve the improvements in the energy intake content X1 and the physiological fatigue level X3 among the four fatigue element indexes, the information encouraging the improvement in life habit such as the meals and the exercise of the user is created.

With the health type of the balance attention type C and the maintenance-of-status-quo type D, information indicative of the good health state is stored in the action improvement information.

With the health type of the balance attention type C, since there is a risk of degenerating the health state, information encouraging the improvement in poorest fatigue element index is stored in the action improvement information. Meanwhile, with the health type of the maintenance-of-status-quo type D, since both the level of elimination of fatigue Z and the fatigue balance B are excellent, information encouraging the maintenance of status quo of the life habit is stored in the action improvement information.

The action improvement information stores the respective evaluation values of the level of elimination of fatigue Z, the fatigue balance B, and the four fatigue element indexes X1 to X4. It should be noted that the action improvement processing unit 25 may represent both the level of elimination of fatigue Z and the fatigue balance B in points and store the total point in the action improvement information.

As described above, the action improvement processing unit 25 uses the respective fatigue element indexes X1 to X4 for each health type to create the action improvement information and cause the storage unit 24 to store the action improvement information. Thus, the action improvement processing unit 25 ensures grasping the increasing trend or the decreasing trend of the fatigue of the user in accordance with the four fatigue element indexes X1 to X4. Accordingly, the action improvement processing unit 25 can propose the improvement in life habit to the user taking the health state in the future into consideration.

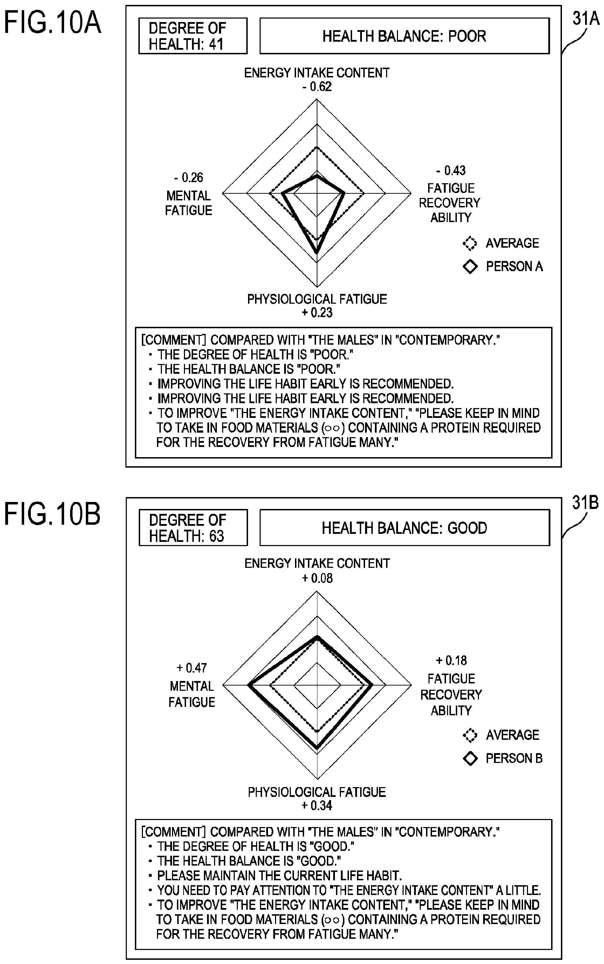

FIG. 10A is a diagram illustrating one example of a display image displayed in the information display terminal 3 on the basis of the action improvement information. FIG. 10B is a diagram illustrating another example of the display image displayed in the information display terminal 3 on the basis of the action improvement information.

In this example, the information display terminal 3 displays the level of elimination of fatigue Z as a degree of health and displays the fatigue balance B as the health balance. Further, the information display terminal 3 normalizes the values of the fatigue element indexes such that the average value of the statistical data becomes "0" and the upper limit value and the lower limit value become "+2" and "-2," respectively for each fatigue element index and displays the normalized values of the fatigue element indexes.

FIG. 10A illustrates a display image 31A in the information display terminal 3 in the case where the user is determined as the early improvement type A.

In the display image 31A, since the evaluation value of the degree of health is poor, 41, and the health balance is poor as well, the comment field describes advice encouraging the early improvement in life habit and the specific reform measures for the energy intake content X1 poorest among the four fatigue element indexes as the action improvement information.

Since the averages illustrated in the radar chart are corrected in accordance with the attribute information indicative of the age and the sex of the user by the information processing device 2, the comment field in the display image 31A describes the results through the comparison with males in contemporary.

FIG. 10B illustrates a display image 31B in the information display terminal 3 when the user is determined as the maintenance-of-status-quo type D.

The display image 31B shows a good evaluation value of the degree of health, 63, and good health balance as well. In view of this, the comment field describes the advice encouraging the maintenance of status quo of the life habit, a notice to the poorest energy intake content X1, and the specific reform measures for the energy intake content X1 as the action improvement information.

Thus, the information display terminal 3 displays the images to encourage the improvement in living on the basis of the action improvement information created for each health type of the user. Accordingly, the user can know the reform measures for the life habit together with can grasping the good and bad of the life habit of himself/herself. This ensures improving the living activity of the user.

It should be noted that while in this embodiment, the four fatigue element indexes X1 to X4 by the radar chart are displayed, the information display terminal 3 may display the fatigue element indexes X1 to X4 in accordance with, for example, a bar chart, a pie chart, or a band graph. While this embodiment describes the example of displaying the action improvement information by the information display terminal 3, the notifying unit 27 in the information processing device 2 may display the action improvement information illustrated in FIG. 9, FIG. 10A, and FIG. 10B.

While in this embodiment, the average value of the statistical data for the determinations whether the fatigue element index is good or bad is used for each fatigue element index, measures of central trend such as frequent value and median of the statistical data may be used as ideal value. Alternatively, a target value aimed by the user himself/herself, a target value settled by the company of the user, or a similar value may be used.

With this embodiment, while the action improvement processing unit 25 uses the above-described four fatigue element indexes X1 to X4 to calculate the level of elimination of fatigue Z, the action improvement processing unit 25 may correct the level of elimination of fatigue Z in accordance with an element index other than the four fatigue element indexes X1 to X4.

Specifically, in physique information such as a Body Mass Index (BMI) of the user obtained by the body composition meter of the biological information detection device 1 or a similar meter, the action improvement processing unit 25 may increase the correction value as a divergence from the average value of the physique information in the attribute to which the user belongs becomes large and may add the correction value to the level of elimination of fatigue Z. Thus, the correction of the level of elimination of fatigue Z in accordance with the physique information of the user ensures obtaining the level of elimination of fatigue Z taking the influence given to the degree of elimination of fatigue by the physique of the user into consideration.

The following describes actions and effects of the first embodiment of the present invention.

With this embodiment, the information processing device 2 includes the measured information obtaining unit 22 and the operating unit 21, which constitute the obtaining means to obtain the life habit information on the life habit of the user. The information processing device 2 includes the fatigue element index computing unit 23 that uses the life habit information to compute at least one element index of the fatigue element indexes X1 to X4 regarding the fatigue accumulation of the user. Further, the information processing device 2 includes the action improvement processing unit 25 that determines whether the improvement in living activity of the user is required or not on the basis of the element indexes.

The above-described fatigue element indexes X1 to X4 are the indexes with which whether the fatigue accumulating to the user is in the increasing trend or in the decreasing trend can be grasped. For example, in the case where at least one fatigue element index indicates an extremely small value, it can be seen that the user is in an abnormal fatigue state and therefore is possibly likely to have the chronic health problem.

Accordingly, the use of at least the one element index among the fatigue element indexes X1 to X4 ensures grasping the fatigue trend of the user; therefore, the person who has the chronic health problem can improve the living activity.

With this embodiment, like the process at Step S421 described in FIG. 6, the action improvement processing unit 25 changes the fatigue element indexes X1 to X4 according to the attribute information on the user. Thus, the use of the attribute information such as the age, the sex, and the occupation of the user by which the standard values of the fatigue element indexes vary ensures appropriately determining the necessity of the improvement in living activity taking the physical property, the labor environment, and a similar factor of the user into consideration. This allows presenting a realistic plan for the improvement in living activity reasonable for the user.

It should be noted that, to determine the necessity of the improvement in living activities, the action improvement processing unit 25 may change, for example, the total threshold Th1, which is described at Step S423 in FIG. 6, or the balance threshold Th2, which is described at Step S433 in FIG. 7, as the determination thresholds for comparison with the parameters for the fatigue element indexes according to the attribute information. In this case as well, the realistic determination taking the physical property, the labor environment, and a similar factor of the user into consideration is possible. It should be noted that the parameters regarding the fatigue element indexes include, for example, the level of elimination of fatigue Z, the fatigue balance B, and the fatigue element indexes themselves.

With this embodiment, the fatigue element index computing unit 23 computes the accumulation element indexes X3 and X4 contributing to the fatigue accumulation of the user and the recovery element indexes X1 and X2 contributing to the recovery from fatigue of the user as the fatigue element indexes. The calculations of both the accumulation element indexes X3 and X4 and the recovery element indexes X1 and X2 make the accurate determination whether the fatigue of the user is in the increasing trend or not possible. Additionally, the detailed reform measures for living activity can be provided.

With this embodiment, the action improvement processing unit 25 mutually adds the accumulation element indexes X3 and X4 to the recovery element indexes X1 and X2 to calculate the level of elimination of fatigue Z correlated with the disorder of life habit. The action improvement processing unit 25 determines whether the improvement in life habit is required or not on the basis of the magnitude of the calculated level of elimination of fatigue Z.

By thus mutually adding the accumulation element indexes X3 and X4 to the recovery element indexes X1 and X2 allows estimating whether the fatigue of the user is steady in the increasing trend or not. Accordingly, the action improvement processing unit 25 can determine the presence/absence of the chronic health problem caused by the disorder of life habit. Therefore, the action improvement processing unit 25 can precisely determine whether the improvement in life habit is required or not.

With this embodiment, as described at Step S422 in FIG. 6, the action improvement processing unit 25 weights the accumulation element indexes X3 and X4 according to the degrees of contribution of the accumulation element indexes X3 and X4 contributing to the fatigue of the user. Further, the action improvement processing unit 25 weights the recovery element indexes X1 and X2 according to the degrees of contribution of the recovery element indexes X1 and X2 contributing to the fatigue of the user. This allows the action improvement processing unit 25 to accurately detect the level of elimination of fatigue Z, ensuring the precise determination whether the improvement in life habit is required or not.

With this embodiment, the operating unit 21 or the measured information obtaining unit 22 obtains at least one of the sex, the age, and the occupation of the user as the attribute information of the user. The action improvement processing unit 25, as described at Step S421 in FIG. 6, changes the respective coefficients a1 to a4 with which the respective fatigue element indexes X1 to X4 are weighted according to the attribute information.

Thus, since the respective fatigue element indexes X1 to X4 are weighted, circumstances such as the physical property and the labor environment of the user can be reflected in detail to the level of elimination of fatigue Z. Accordingly, the action improvement processing unit 25 can further accurately detect the level of elimination of fatigue Z.

With this embodiment, as illustrated in FIG. 7, the action improvement processing unit 25 obtains the fatigue balance B regarding the plurality of fatigue element indexes X1 to X4 to determine whether the improvement in life habit is required or not on the basis of the fatigue balance B. For example, the action improvement processing unit 25 multiplies each of the plurality of fatigue element indexes X1 to X4 by the other fatigue element indexes to calculate the variance value of the respective multiplied values as the fatigue balance B.

For example, the poor fatigue balance B possibly also degenerates the fatigue element index indicative of the good state. In view of this, obtaining the fatigue balance B by the action improvement processing unit 25 ensures grasping the fatigue trend of the user in the future. Furthermore, specifying the fatigue element index indicative of the poor state makes it possible to predict the trend of increase or decrease of the fatigue element index indicative of the good state. Thus, the use of the fatigue balance B ensures further appropriately providing the reform measures for the life habit.

With this embodiment, as illustrated in FIG. 8 and FIG. 9, the action improvement processing unit 25 determines whether the improvement in life habit is required or not on the basis of the two parameters, the level of elimination of fatigue Z and the fatigue balance B. Accordingly, the action improvement processing unit 25 can precisely determine the necessity of the improvement in life habit taking the trend of the health state of the user in the future into consideration.

With this embodiment, the information processing device 2 includes the notifying unit 27, which notifies the action improvement information to encourage the improvement in living activity on the basis of the determination results by the action improvement processing unit 25. The action improvement processing unit 25 creates the action improvement information on the basis of the fatigue element indexes X1 to X4. The action improvement information includes, for example, as illustrated in FIG. 10A and FIG. 10B, the evaluation value of the level of elimination of fatigue Z, the evaluation value of the fatigue balance B, the evaluation values of the fatigue element indexes X1 to X4, and the advice describing the living activity required to improve these evaluation values.

Thus, the notifying unit 27 notifies the action improvement information as illustrated in FIG. 9, FIG. 10A, and FIG. 10B; therefore, the user can grasp good and bad of the living activity of himself/herself, thereby ensuring improving the living activity of the user.

Further, with this embodiment, the notifying unit 27 notifies the reform measures regarding the poorest fatigue element index among the plurality of fatigue element indexes X1 to X4. This allows the user to effectively improve the life habit.

### (Second Embodiment)

While in the above-described embodiment, the action improvement information for the person with the good level of elimination of fatigue Z is displayed as well, the information processing device 2 of the second embodiment of the present invention displays the action improvement information only for the person with the poor level of elimination of fatigue Z.

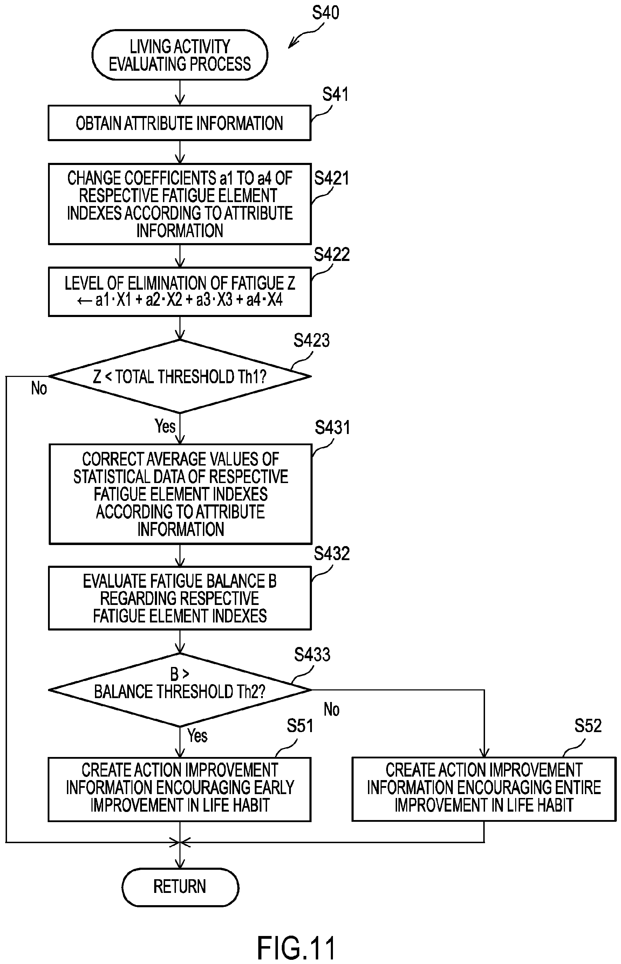

FIG. 11 is a flowchart illustrating a process procedure example regarding the living activity evaluating process at Step S40 of the embodiment.

The process at Step S40 in this embodiment includes a part of processes Steps S421 to S423 in the total fatigue level determining process illustrated in FIG. 6 and a part of the processes Steps S431 to 433 in the fatigue balance determining process illustrated in FIG. 7.

Further, the process at Step S40 includes the processes at Steps S51 and S52 instead of the processes at Steps S44 and S45 illustrated in FIG. 5. This embodiment describes only the processes at Steps S51 and S52 in detail.

At Step S51, when the level of elimination of fatigue Z falls below the total threshold Th1 and the fatigue balance B exceeds the balance threshold Th2, the action improvement processing unit 25 creates the action improvement information encouraging the early improvement in life habit. That is, when both the level of elimination of fatigue Z and the fatigue balance B are poor, the action improvement processing unit 25 proposes the reform measures for the life habit by which the user recovers from the fatigue early such that the fatigue state is not further degenerated.

At Step S52, when the level of elimination of fatigue Z falls below the total threshold Th1 and the fatigue balance B is equal to or less than the balance threshold Th2, the action improvement processing unit 25 creates the action improvement information encouraging the overall improvement in life habit. That is, when the level of elimination of fatigue Z is poor and the fatigue balance B is good, the action improvement processing unit 25 proposes the improvement from one that the user easily tackles with among, for example, the meal, the activities, the sleeping, and the environment.

When the process at Step S51 or S52 is terminated, a sequence of the process procedures of the living activity evaluating process of the embodiment are terminated.

Thus, the second embodiment of the present invention, similar to the first embodiment, can improve the life habit of the person who has the low level of elimination of fatigue Z, that is, the person whose life habit is disordered. Furthermore, the use of the fatigue balance B can determine a degree of urgency of the improvement in life habit; therefore, this embodiment allows proposing the reform measures reasonable for the user and adding the future fatigue condition to the user.

### (Third Embodiment)

While in the above-described embodiments, the variation in the four fatigue element indexes X1 to X4 is evaluated, the information processing device 2 of the third embodiment of the present invention encourages the improvement in living activity of the user according to the relationship between the recovery element indexes and the accumulation element indexes.

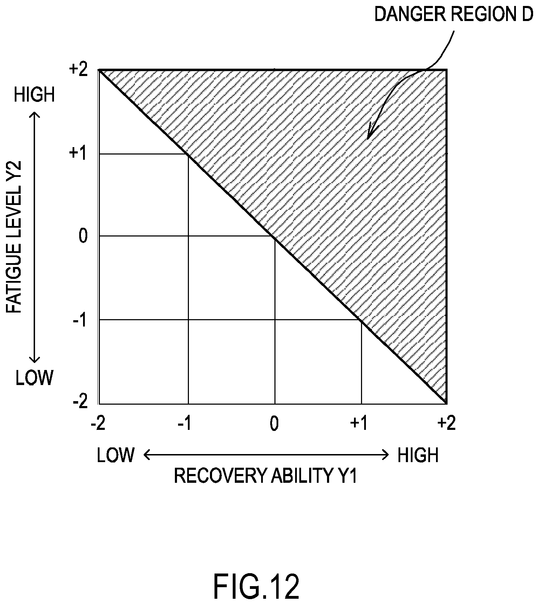

FIG. 12 is a diagram describing a relationship between a recovery ability Y1 and a fatigue level Y2 of the user. The recovery ability Y1 and the fatigue level Y2 are indexes normalized such that the recovery ability Y1 and the fatigue level Y2 can be uniformly treated mutually.

The recovery ability Y1 is calculated on the basis of at least one of the recovery element indexes among the energy intake content X1 and the fatigue recovery ability X2 as the recovery element indexes. The fatigue level Y2 is calculated on the basis of at least one of the accumulation element indexes among the physiological fatigue level X3 and the mental fatigue level X4 as the accumulation element indexes. As the recovery ability Y1 becomes high, the fatigue level Y2 becomes small.

A danger region D is a region indicative of a state in which the fatigue level Y2 exceeds a threshold of the fatigue level Y2, that is, although the user has the recovery ability Y1, the fatigue level Y2 is not solved. It should be noted that, usually, the higher the recovery ability Y1 is, the lower the fatigue level Y2 is. As a determination criterion to determine such state where the relationship between the recovery ability Y1 and the fatigue level Y2 is inconsistent, the threshold for the fatigue level Y2 is set, and this threshold becomes small as the recovery ability Y1 increases. This danger region D is a region in which there is a possibly of accumulating the fatigue to the extent of resulting in unwell such as the user feeling sleepy suddenly due to the chronic fatigue. The danger region D of this embodiment is the region in which the sum of the recovery ability Y1 and the fatigue level Y2 exceeds 0 (zero). Therefore, in the health state where an intersection point between the recovery ability Y1 and the fatigue level Y2 is included in the danger region D, driving of a moving vehicle such as an automobile, an airplane, and a ship is preferably avoided.

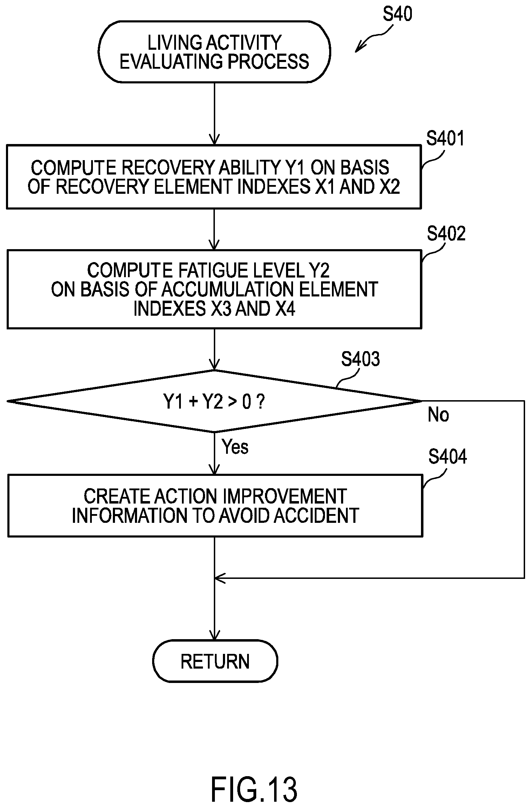

FIG. 13 is a flowchart illustrating a process procedure example regarding the living activity evaluating process at Step S40 of the embodiment.

At Step S401, the action improvement processing unit 25 applies the energy intake content X1 and the fatigue recovery ability X2 to the predetermined regression formula or the predetermined correspondence table to calculate the recovery ability Y1.

At Step S402, the action improvement processing unit 25 applies the physiological fatigue level X3 and the mental fatigue level X4 to the predetermined regression formula or the predetermined correspondence table to calculate the fatigue level Y2.

At Step S403, the action improvement processing unit 25 determines whether the sum of the recovery ability Y1 and the fatigue level Y2 exceeds 0 or not. That is, the action improvement processing unit 25 determines whether the health state of the user is in the chronic fatigue state or not. With the sum of the recovery ability Y1 and the fatigue level Y2 equal to or less than 0, the action improvement processing unit 25 terminates the living activity evaluating process.

At Step S404, when the sum of the recovery ability Y1 and the fatigue level Y2 exceeds 0, since this state is equivalent to the danger region D, which indicates that the health state of the user is in the chronic fatigue state, the action improvement processing unit 25 creates the action improvement information to avoid an accident. For example, in the case where the occupation of the user is a driver, the action improvement processing unit 25 creates warning information indicative of quitting the driving of the automobile as the action improvement information.

When the process at Step S404 is terminated, the process returns to FIG. 3 and the process advances to Step S50. For example, when the occupation of the user is the driver, the information processing device 2 transmits the action improvement information to a car navigation system constituting the information display terminal 3. At Step S50, the car navigation system displays the warning information indicative of quitting the driving of the automobile or a similar vehicle.

Specifically, when the sum of the recovery ability Y1 and the fatigue level Y2 exceeds 0, the improvement information transmitting unit 26 extracts identification information of the user from an identification information table in the storage unit 24 and transmits the warning information to a terminal with a destination indicated by the identification information. Afterwards, the warning information is forwarded to the car navigation system in accordance with the destination of the terminal via the network 101 and is received by the car navigation system.

With the third embodiment of the present invention, the action improvement processing unit 25 uses the accumulation element indexes X3 and X4 and the recovery element indexes X1 and X2 to achieve the improvement in living activity such as the driving of the automobile of the user in the chronic fatigue state. This ensures avoiding a car accident and a similar accident.

It should be noted that while this embodiment displays the warning information when the fatigue level Y2 exceeds the recovery ability Y1, at least one piece of information among the fatigue information indicative of the evaluation values of the four fatigue element indexes X1 to X4, the life habit information, and the biological information may be analyzed such that specific reform measures for the living activity is proposed.

For example, when the energy intake content X1 is lower than the average value, the improvement information transmitting unit 26 may transmit command information that instructs the car navigation system to display a convenience store, an eating place, and a similar place nearby the user himself/herself together with the action improvement information of taking a meal required for the recovery from fatigue.

Alternatively, when the fatigue recovery ability X2 is lower than the average value, the improvement information transmitting unit 26 may transmit the warning information indicative of encouraging quitting the long-time driving of the automobile as the action improvement information or may transmit the warning information indicative of encouraging quitting the driving at every predetermined time to the car navigation system.

While the embodiments of the present invention are described above, the above-described embodiments describe merely a part of application examples of the present invention and the gist does not limit the technical scope of the present invention to the specific configuration of the embodiment.

For example, when the occupation indicated by the attribute information is the driver, the improvement in meal is important from the aspect of avoiding the car accident. Therefore, when it is determined that the fatigue balance B is poor, the action improvement processing unit 25 may encourage the improvement in meal in preference to the improvements in sleeping and exercise. Specifically, for example, improving the nutrient balance, not skipping breakfast, and insufficient nutrient required for the recovery from fatigue or the muscle repair are presented.

Additionally, even when it is determined that the fatigue balance B is poor, the case where the fatigue of the user is improved is assumed. For example, in the case where both of the energy intake content X1 and mental fatigue level X4 are good and both of the fatigue recovery ability X2 and physiological fatigue level X3 are poor, there is a possibility that the fatigue recovery ability X2 and the physiological fatigue level X3 are improved. In the case where both of the energy intake content X1 and fatigue recovery ability X2 are good and both of the physiological fatigue level X3 and mental fatigue level X4 are poor, there is a possibility that the blood pressure variation is improved and the mental fatigue level X4 is improved.

Thus, even if the fatigue balance B is determined as poor, there may be a case where the fatigue of the user is improved. Accordingly, when it is determined that the fatigue balance B is poor, the action improvement processing unit 25 may additionally change the reform measures according to the combination of the poor fatigue element index and the good fatigue element index. Alternatively, the action improvement processing unit 25 may correct the evaluation value of the fatigue balance B according to the combination of the poor fatigue element index and the good fatigue element index. This allows proposing the further appropriate reform measures for the living activity to the user.

While in the embodiments, the four fatigue element indexes X1 to X4 Z to obtain the fatigue balance B is used, the action improvement processing unit 25 may obtain a difference between the energy intake content X1 and the physiological fatigue level X3, a difference between the fatigue recovery ability X2 and the mental fatigue level X4, or a similar value as the fatigue balance B. In this case as well, it is possible to grasp the trend of the fatigue state of the user in the future.

The biological information detected by the biological information detection device 1 may include biological indexes such as fat percentages of the entire body and at each site in the entire body, a fat mass, a fat-free mass, a muscle mass, a visceral fat mass, a visceral fat level, a visceral fat area, a subcutaneous fat mass, a basal metabolic rate, a bone mass, a body moisture content, BMI, an intracellular fluid volume, and an extracellular fluid volume. The action improvement information may be additionally created in accordance with such biological information.

### DESCRIPTION OF REFERENCE SIGNS

- 1: biological information detection device
- 2: information processing device
- 3: information display terminal
- 21: operating unit (obtaining means)
- 22: measured information obtaining unit (obtaining means)
- 23: fatigue element index computing unit (computing means)
- 24: storage unit (program)
- 25: action improvement processing unit (determining means)
- 27: notifying unit (notifying means)
- S20 to S40: (obtaining step, computing step, and determining step)

## Claims

1. An information processing device (2) comprising:
obtaining means (21, 22) configured to obtain life habit information on a life habit of a user;
computing means (23) configured to compute an element index (X1 to X4, Z, B) regarding fatigue accumulation of the user in accordance with the life habit information obtained by the obtaining means (21, 22); and
determining means (25) configured to determine whether an improvement in living activity of the user is required or not on the basis of the element index (X1 to X4, Z, B) computed by the computing means (23).

2. The information processing device (2) according to claim 1, wherein:
the obtaining means (21, 22) is configured to further obtain attribute information on the user, and
the determining means (25) is configured to change the element index (X1 to X4, Z, B) or a determination threshold (Th1, Th2, Min) for the element index (X1 to X4) in accordance with the attribute information.

3. The information processing device (2) according to claim 1 or 2, wherein
the computing means (23) is configured to compute an accumulation element index (X3, X4) and a recovery element index (X1, X2) as the element indexes, the accumulation element index (X3, X4) contributing to the fatigue accumulation of the user, the recovery element index (X1, X2) contributing to a recovery from fatigue of the user.

4. The information processing device (2) according to claim 3, wherein
the determining means (25) is configured to:
calculate a level of elimination of fatigue (Z) regarding the life habit in accordance with the accumulation element index (X3, X4) and the recovery element index (X1, X2), and
determine whether the improvement in living activity is required or not on the basis of the level of elimination of fatigue.

5. The information processing device (2) according to claim 3 or 4, wherein
the determining means (25) is configured to weight the accumulation element index (X3, X4) and the recovery element index (X1, X2) in accordance with respective levels of contribution of the accumulation element index (X3, X4) and the recovery element index (X1, X2) that are contributing to the fatigue of the user.

6. The information processing device (2) according to claim 5, wherein:
the obtaining means (21, 22) is configured to obtain at least one of a sex, an age, and an occupation of the user as the attribute information, and
the determining means (25) is configured to change the weighting (a1 to a4) in accordance with the attribute information.

7. The information processing device (2) according to any one of claim 1 to 6, wherein
the determining means (25) is configured to:
obtain a balance (B) between a plurality of the element indexes (X1 to X4) regarding the fatigue accumulation, and
determine whether the improvement in living activity is required or not on the basis of the balance (B).

8. The information processing device (2) according to claim 7, wherein
the determining means (25) is configured to:
add each of the plurality of element indexes (X1 to X4) to calculate the level of elimination of fatigue (Z) caused by the life habit, and
determine whether the improvement in living activity is required or not on the basis of the balance (B) and the level of elimination of fatigue (Z).

9. The information processing device (2) according to any one of claim 1 to claim 8, further comprising
notifying means (27) configured to notify improvement information encouraging the improvement in living activity on the basis of a determination result by the determining means (25).

10. A non-transitory computer-readable storage medium (24) that records a program to cause a computer configured to process life habit information on a life habit of a user to execute:
an obtaining step (S20) of obtaining the life habit information;
an computing step (S30) of computing an element index (X1 to X4, Z, B) regarding fatigue accumulation of the user in accordance with the life habit information obtained by the obtaining; and
a determining step (S40) of determining whether an improvement in living activity of the user is required or not on the basis of the element index (X1 to X4, Z, B) computed by the computing.

11. An information processing method comprising:
an obtaining step (S20) of obtaining life habit information on a life habit of a user;
an computing step (S30) of computing an element index (X1 to X4, Z, B) regarding fatigue accumulation of the user in accordance with the life habit information obtained by the obtaining; and
a determining step (S40) of determining whether an improvement in living activity of the user is required or not on the basis of the element index (X1 to X4, Z, B) computed by the computing.
